(19)

**Europäisches Patentamt**
**European Patent Office**
**Office européen des brevets**

(11) **EP 2 893 948 A1**

(12) **EUROPEAN PATENT APPLICATION**

(43) Date of publication:
**15.07.2015 Bulletin 2015/29**

(51) Int Cl.:
***A61M 16/06*** *(2006.01)*    ***A61M 16/00*** *(2006.01)*

(21) Application number: **14382007.4**

(22) Date of filing: **10.01.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **Fundació Institut d'Investigació Biomèdica de Bellvitge (IDIBELL)**
**08907 L'Hospitalet de Llobregat (ES)**
• **UNIVERSITAT AUTONOMA DE BARCELONA**
**08193 Bellaterra (ES)**
• **Centro de Investigación Biomédica en Red (CIBER)**
**28029 Madrid (ES)**

(72) Inventors:
• **Molina Molina, Maria**
**08907 L'Hospitalet de Llobregat (ES)**
• **Rosell Gratacòs, Antoni**
**08907 L'Hospitalet de Llobregat (ES)**
• **Aguiló, Jordi**
**08193 Bellaterra (ES)**

(74) Representative: **ZBM Patents - Zea, Barlocci & Markvardsen**
**Plaza Catalunya, 1**
**08002 Barcelona (ES)**

(54) **Methods and systems for providing oxygen to a patient**

(57)    System for providing oxygen to a patient, comprising a sensing device (41) having an oxygen sensor (41) for obtaining one or more measurements of a level of oxygen in patient's blood, an oxygen provider (45) for providing oxygen to the patient, and a control unit (40). The control unit (40) is configured to receive the level of oxygen in blood from the oxygen sensor (41), and to determine a parameter representative of oxygen insufficiency by comparing the level of oxygen in blood with an oxygen level threshold. The control unit (40) is further configured to determine an oxygen need depending at least on the parameter representative of oxygen insufficiency, and to cause the oxygen provider (45) to continuously provide the oxygen need to the patient until the oxygen need is determined to be substantially null.

Figure 4

**Description**

The present invention relates to methods of providing oxygen to a patient, and to systems suitable for carrying out such methods.

BACKGROUND ART

Patients suffering from different types of lung diseases may occasionally or regularly have low levels of oxygen in their blood and may require extra oxygen (i.e. an oxygen supplement).

Adults and children with obstructive lung diseases such as chronic obstructive pulmonary disease (COPD), cystic fibrosis (CF), bronchiectasis, bronchiolitis or bronchopulmonary dysplasia (BPD), interstitial lung diseases (ILD) such as idiopathic pulmonary fibrosis (IPF), and vascular lung diseases such as primary pulmonary hypertension may require such a treatment.

Chronic obstructive pulmonary disease (COPD) is a type of obstructive lung disease characterized by chronic airflow limitation with a decrease of forced respiratory volume at the first second (FEV1). It typically worsens over time, with the main symptoms including shortness of breath and air trapping, cough, and sputum production. Supplemental oxygen is recommended in those patients with low oxygen levels at rest.

Idiopathic pulmonary fibrosis (IPF) is the most lethal chronic ILD (mean survival of 2 - 4 years from diagnosis). IPF and other fibrotic ILD are characterized by a progressive decline in lung function (forced vital capacity-FVC and total lung volume-TLC) because of a progressive scarring such that they tend to lose elasticity, which may make the inhalation increasingly more difficult. The scarring is permanent once it has developed. Treatment options for pulmonary fibrosis may be regarded as non-curative and are rather limited (anti-fibrotic drugs, immunosupressors, glucocorticoids or lung transplant). Oxygen supplementation is recommended to improve the quality of life and exercise tolerance.

For different kinds of patients suffering from different lung diseases, the amount of oxygen required may be very different. An oxygen requirement may be substantially continuous i.e. at all times, or very punctual. For all patients, an extra oxygen supplement may protect the patient's body against the effects of low levels of oxygen and, therefore, help the patient to work better, to remain more active, to be more comfortable and/or to avoid further progression of the disease, etc.

Systems are known for providing oxygen to a patient based on an uninterrupted supply of an oxygen flow. These systems may cause drying up and irritation of the patient's nostrils due to the continuity of the provision of oxygen. Besides, the consumption of oxygen by these systems may be relatively large and, consequently, inefficient. This inefficiency can cause e.g. high healthcare costs, the necessity of the patient to be "connected" to large (i.e. non-portable) machinery with sources of oxygen, etc.

Other known systems are based on having inhalation sensors in order to detect when the patient is inhaling and to provide oxygen only when the patient is inhaling. Some of these systems further comprise other types of sensors for sensing other physiological parameters, such as e.g. patient's physical activity, for restricting even more the periods of oxygen dispensation depending on said other sensed parameter(s).

For example, US2002112726 discloses a system/method of providing oxygen to a patient based on detecting when the patient is inhaling and measuring the oxygen in blood of the patient. In this system/method, when the oxygen in blood is considered insufficient, an oxygen dose is provided to the patient when the patient is inhaling. This system may achieve, indeed, a rather efficient consumption of oxygen, since the provision of oxygen is restricted to particularly the period of inhalation. This system may however be rather useless when the inhalation cannot be detected reliably. This can be the case for a group of patients.

In patients suffering from idiopathic pulmonary fibrosis (IPF) and other fibrotic interstitial lung diseases (ILD), inhalation may be undetectable or only be detectable in an unreliable manner. If the inhalation is not detected, no oxygen is supplied. This can have serious consequences when a patient is in need of oxygen. Therefore, systems/methods such as those of US2002112726 may be useless when used for providing oxygen to patients with IPF and/or other fibrotic ILD, due to the fact that inhalation may not be sufficiently well detected by the corresponding inhalation sensors.

SUMMARY OF THE INVENTION

It is an object of the present invention to provide methods and systems for (controllably) supplying oxygen to a patient, in particular to patients suffering from fibrotic interstitial lung diseases, resolving at least some of the aforementioned problems.

In a first aspect, the invention provides a method of providing oxygen to a patient, comprising determining a level of oxygen in patient's blood, and determining a parameter representative of oxygen insufficiency by comparing the level of oxygen in the patient's blood with an oxygen level threshold (previously established). The method further comprises determining an oxygen need depending at least on the parameter representative of oxygen insufficiency, and continuously providing the oxygen need to the patient until the oxygen need is determined to be substantially null.

In this method, "continuously providing" means that the provision of oxygen is not restricted to when the patient is inhaling, so no inhalation detector or sensor is required. Instead, the amount of oxygen to be supplied only depends on a (continuously) determined oxygen need depending on the amount of oxygen in the patient's blood. When an oxygen need is detected, extra oxygen is continuously provided to the patient until the oxygen need is determined to be substantially satisfied because e.g. the oxygen level in blood is determined to be high enough.

This proposed method is aimed at being more suitable for patients suffering from idiopathic pulmonary fibrosis (IPF) and/or other interstitial lung diseases (ILD), for which detection of inhalation may be undetectable or at least not be detected in a sufficiently reliable manner. Even though inhalation detectors are avoided, oxygen is "rationally" provided (only when needed) to the patient, which may prevent the inconveniences associated with an uninterrupted oxygen supply. This proposed approach may also permit a reduced consumption of oxygen, which may cause e.g. lower healthcare costs and the feasibility of using an oxygen source (oxygen tank or oxygen generator) of reduced size, which may thus be portable and thus have an advantageous impact on the usability of the device.

The expression "level of oxygen in patient's blood" refers to an indicator of how much oxygen there is in the patient's blood. This indicator may be e.g. a percentage, a scale value (e.g. very-low, low, medium, high, very-high) which may be relative to an absolute reference or referred to values and needs of the specific patient, or an absolute oxygen value, etc. The expression "parameter representative of oxygen insufficiency" refers to an indicator of to which extent the oxygen level in blood is considered insufficient for the patient to be in a good condition. This indicator may also be e.g. a percentage (of insufficiency), a scale value, or an absolute value (of insufficiency), etc.

For example, in the case of these indicators being percentages, a "level of oxygen in patient's blood" of 87% may be considered insufficient when compared to an "oxygen level threshold" of e.g. 90%, in which case a "parameter representative of oxygen insufficiency" of 3% could be determined.

The expression "oxygen need" refers to an indicator of how much oxygen is required for the patient to achieve a good condition. This indicator may be e.g. an absolute amount (e.g. a volume) of oxygen. In the previous example, the determined "oxygen insufficiency" of 3% may be considered to require an "oxygen need" of e.g. 1 litre/minute of oxygen. In this case a continuous flow of oxygen may be provided to the patient until this oxygen need is determined to have been satisfied. The oxygen need may be evaluated substantially continuously (e.g. at a predetermined frequency of a second, or a few seconds) and adapted accordingly. The oxygen need may be determined satisfied if e.g. an oxygen insufficiency of 0% or near 0% is determined in a new iteration of the process.

In some embodiments, the method may comprise determining a parameter representative of a patient's activity, such that the oxygen need may be determined at least partly based also on this parameter representative of patient's activity. This may permit determining the oxygen need in a more reliable manner according to the patient's status. It may also permit determining an oxygen need before an actual oxygen insufficiency arises. An oxygen supply system may anticipate an imminent oxygen insufficiency and in general be quicker to react. The parameter representative of patient's activity may be defined in terms of e.g. absolute values (of e.g. acceleration along one or more axes provided by an accelerometer), scale values (e.g. lying, sitting, walking, rapid walking, running, etc.), and so on.

Depending on the determined patient's activity, the abovementioned oxygen level threshold may be varied to cause a variation of oxygen insufficiency, and thus indirectly the oxygen need of the patient. In some other cases, the oxygen level threshold may not be varied and, instead, the oxygen need may be determined directly further depending on the patient's activity. In still other examples, a variation of the oxygen level threshold and a "direct" variation of the oxygen need may be combined together to obtain the oxygen need. For example, if a high activity is detected, the oxygen level threshold may be accordingly increased (for causing an "indirect" increase of the oxygen need) and/or the oxygen need may be directly increased further depending on the determined level of activity. That is, the oxygen level threshold may be related to the patient itself and also to its present status i.e. walking, running, affected by influenza, etc.

According to various embodiments, the method may comprise determining a parameter representative of a patient's skin temperature, such that the oxygen need may be determined at least partly based on this parameter representative of the patient's skin temperature. This optional feature is based on the consideration that a higher skin temperature may indicate e.g. that the patient is making an effort and/or has fever, etc. In this case, an increased oxygen amount may be accordingly provided to the patient. The parameter representative of patient's skin temperature may be e.g. a percentage of difference with respect to e.g. a reference temperature of 37.0 °C, a scale value (e.g. very high, high, normal, etc.), an absolute value (of skin temperature), etc.

A logic similar to the one previously commented with respect to patient's activity may also be applied when considering the skin temperature for determining the oxygen need. Therefore, in some examples, the amount of oxygen to be provided to the patient may be varied further depending on the temperature of the patient's skin indirectly (by e.g. varying the oxygen level threshold depending on the oxygen in blood, skin temperature, and possibly other optional parameters) or directly (by e.g. defining direct relations between oxygen in blood, skin temperature, and possibly other optional parameters, and how much to vary the oxygen amount). Alternatively, the amount of oxygen to be provided may be determined by combining said indirect and direct approaches.

In some embodiments, the method may comprise determining a parameter representative of a patient's skin impedance, such that the oxygen need may be determined at least partly based also on this parameter representative of the patient's skin impedance. This is based on the fact that variations of the skin impedance may indicate that the patient is sweating, which may mean that the patient is making an effort, is feverish, or is otherwise uncomfortable. This further consideration of the skin impedance may thus make the determination of the oxygen need more reliable. The parameter representative of patient's skin impedance may again be e.g. a percentage (with respect to e.g. an impedance of reference), a scale value (e.g. very high, high, normal, low etc.), or an absolute value (of impedance), etc.

In some examples, the amount of oxygen to be provided to the patient may be varied depending on the impedance of the patient's skin indirectly (by e.g. varying the oxygen level threshold depending on the oxygen in blood, skin impedance, and possibly other optional parameters) or directly (by e.g. defining direct relations between oxygen in blood, skin impedance, and possibly other optional parameters, and how much to vary the oxygen amount accordingly). In alternative examples, the amount of oxygen to be provided may be determined by combining said indirect and direct approaches.

According to some embodiments, the method may further comprise determining a parameter representative of a patient's cardiac frequency, such that the oxygen need may be determined at least partly based also on this parameter representative of the patient's cardiac frequency. A certain cardiac frequency or a variation in the cardiac frequency may mean e.g. that the patient is making an effort. The patient may thus be in need of an extra oxygen dose. The cardiac frequency may increase the reliability of the determination of the oxygen need. The parameter representative of a patient's cardiac frequency may be e.g. a percentage (with respect to e.g. a frequency of reference), a scale value (e.g. very high, high, normal, low, etc.), an absolute value (of e.g. beats per minute), etc.

As in the case of skin impedance, activity and skin temperature, the amount of oxygen to be provided to the patient may be varied depending on the cardiac frequency of the patient indirectly (by changing the oxygen threshold) or directly. In alternative examples, the amount of oxygen to be provided may be determined by combining said indirect and direct approaches.

In some embodiments, the method may further comprise receiving a parameter representative of patient's medical condition, such that the oxygen need may be determined at least partly based also on this parameter representative of patient's medical condition. The patient may have different oxygen needs depending on e.g. the evolution of the respiratory disease suffered by the patient. The current medical condition of the patient may thus be a relevant parameter for determining the oxygen need more reliably. The parameter representative of patient's medical condition may be a percentage (indicating e.g. the progress of the respiratory illness), a scale value (initial stage, advanced stage, final stage, etc.).

In examples of the method, the oxygen need may be varied further depending on the medical condition of the patient indirectly or directly. An indirect variation may be implemented by e.g. varying the oxygen level threshold depending on the medical condition, and possibly other optional parameters. A direct variation may be implemented by e.g. defining direct relations between oxygen in blood, medical condition, and possibly other optional parameters, and how much the oxygen amount has to be varied accordingly. In alternative examples, the oxygen need may be determined by combining said indirect and direct approaches.

In embodiments of the method, determining the oxygen need may comprise using a PID control loop based on at least one of the representative parameters. A PID control comprises calculating an "error" value as the difference between a measured process variable (oxygen level in blood) and a desired set point (oxygen threshold).

A typical PID control may be $O_{2need}(t) = K_p e(t) + K_i \int_o^t e(\tau)d\tau + K_d \frac{de(t)}{dt}$, wherein t is the time or instantaneous time, $\tau$ is a variable of integration, $O_{2need}$ stands for the oxygen need, Kp is a proportional gain constant, $K_i$ is an integral gain constant and $K_d$ is a derivative gain constant, e(t) is the error value as a function of time and is equal to $e(t) = O_{2measured}(t) - O_{2threshold}$.

Simply put, these values can be interpreted in terms of time: P depends on the present error, I on the accumulation of past errors, and D is a prediction of future errors, based on current rate of change. The weighted sum of these three actions is used to adjust the process via a control element such as the position of a control valve.

The proportional, integral and derivative gain constants may be considered fine-tuning parameters. In some examples, one or more of these gain constants may be equal to zero. Herein the term PID control also covers these options although it could alternatively be called PI or PD control (depending on which constant is zero).

In examples wherein other parameters such as e.g. physical activity, skin temperature, skin impedance etc. are used, one or more of the constants may be changed. A PID control would thus adapt the oxygen supply accordingly. Alternatively, depending on these parameters, the oxygen threshold may be varied, so that the error value is affected.

In another aspect, the invention provides a system for supplying oxygen to a patient, comprising a sensing device having an oxygen sensor for obtaining one or more measurements of a level of oxygen in patient's blood, and an oxygen provider for providing oxygen to the patient. The system further comprises a control unit configured to receive the level of oxygen in blood from the oxygen sensor, and to determine a parameter representative of oxygen insufficiency by comparing the level of oxygen in blood with an oxygen level threshold. The control unit is further configured to determine an oxygen need depending at least on the parameter representative of oxygen insufficiency, and to cause the oxygen provider to continuously provide the oxygen need to the patient until the oxygen need is determined to be substantially null.

In some embodiments, the system may further comprise one or more additional sensors for obtaining one or more measurements of one or more parameters representative of patient's activity and/or patient's temperature and/or patient's skin impedance and/or patient's cardiac frequency. Besides, the control unit may be further configured to receive said one or more parameters, and to determine the oxygen need at least partly based also on said one or more received parameters (representative of patient's activity and/or patient's temperature and/or patient's skin impedance and/or patient's cardiac frequency). Therefore, depending on the patient's need and optionally on a medical prescription, the system (including some of the above mentioned sensors) may allow a time-modulated oxygen flux according to e.g. the patient's heart rate.

In embodiments of the system, at least one of the abovementioned further sensors may also be comprised/incorporated in the sensing device. In particular implementations, all the sensors of the system, i.e. the oxygen (in blood) sensor and any other of the optional (further) sensors, may be integrated in the same sensing device. In this case, a completely integrated sensing platform would be provided to be arranged in/on a single point of the patient's body. An aspect of this integrated solution may be that the system may be more comfortable to the patient in comparison with other alternatives having different sensors distributed among different points of the patient's body.

The sensing device may be, in some configurations of the system, a percutaneous sensing device. In this approach, some elements of the sensing device, such as e.g. the optional skin temperature sensor may simply have a surface arranged to be in permanent contact with a part of the skin. Other elements, such as the oxygen (in blood) sensor may in some implementations have a single small extension to reach a suitable (e.g. subcutaneous) point in the patient's body for continuously having blood samples available. Apart from these particularities, any suitable adhesive (strip or tape) may be used to attach the overall sensing device on somewhere the patient's body.

In some examples, the sensing device may be adapted to be arranged in a position of the patient's chest. This particular feature may be especially suitable when e.g. a cardiac frequency sensor is included in the sensing device. In these embodiments, the system may comprise e.g. a belt or the like to be arranged around the patient chest, such that the sensing device is suitably attached to a corresponding area of the patient's chest. Optionally, the aforementioned tape may also be present to improve the attachment of the sensing device to the patient's chest.

In some implementations, the control unit may also be comprised in the sensing device, such that a fully integrated system may be provided if all the sensors of the system are also comprised in the sensing device. This way, uncomfortable connections (by means of e.g. cables) between the control unit and sensors may thus be avoided. In not fully integrated configurations, wireless connections between the control unit and at least some sensors could be present instead of cables. For example, accelerometers may in some implementations be arranged at points of the patient's body (e.g. at extremities) separated from the sensing device, in which case such wireless connections could be advantageously used. In other configurations, the control unit may be a remote control unit which may be wirelessly connected with the sensors of the sensing device.

According to some embodiments of the system, the control unit may be wirelessly connected with the oxygen provider. Since the oxygen provider (and corresponding oxygen tank) may be a separate component from the sensing device, some kind of connection with the control unit is required. This connection can be used for the control unit to send signals to the oxygen provider (indicating e.g. the amount of oxygen to be provided to the patient), and for the oxygen provider to send signals (indicating e.g. its status) to the control unit. Such a wireless connection may permit e.g. avoiding the presence of cables between the control unit and the oxygen provider, so the system may be more comfortable to the patient.

If the control unit is not a remote control unit (i.e. it is arranged e.g. in the sensing device or somewhere attached to the patient's body), the system may comprise a communications device for communicating the "local" control unit with a remote control unit (of e.g. a Hospital information system HIS). This remote control unit may be arranged in a hospital environment, and may comprise relevant medical data and a communications system to interchange data with e.g. one or more systems for providing oxygen to a patient such as the one described before.

This connection between the local and remote control units may be a wireless connection. This way, the local control unit may send to the remote control unit any kind of data, such as e.g. medical data about the patient for its storage by the remote control unit (in e.g. a repository of a HIS), alarm data in case of e.g. determining an abnormal oxygen insufficiency, etc. Also through this connection, the remote control unit (of e.g. a HIS) may send data to the local control unit, such as e.g. data about the medical condition of the patient, which may be used, as commented before, as a parameter to determine the oxygen need.

In configurations of the system having a remote control unit and not a local control unit, the system may comprise a connection (which may be wireless) between the sensors and the remote control unit (such as e.g. the HIS commented before). Taking this into account, all the data (oxygen in blood and possibly other optional parameters) determined/collected by the sensors may be sent to the HIS, such that the HIS may determine the corresponding oxygen insufficiency and need. Then, the HIS may send corresponding signals to cause the oxygen provider to continuously provide the oxygen need to the patient until the oxygen need is determined to be null or almost null.

In embodiments of the system, the oxygen provider may be a portable oxygen provider, such that the patient may move freely and, therefore, a patient's autonomy may be enhanced.

BRIEF DESCRIPTION OF THE DRAWINGS

Particular embodiments of the present invention will be described in the following by way of non-limiting examples, with reference to the appended drawings, in which:

Figure 1 is a flow chart schematically illustrating an embodiment of one of the methods provided by the invention;

Figure 2 is a flow chart schematically illustrating another embodiment of one of the methods provided by the invention;

Figure 3 is a flow chart schematically illustrating yet another embodiment of one of the methods provided by the invention;

Figure 4 is a block diagram schematically illustrating an embodiment of the system provided by the invention; and

Figure 5 schematically illustrates a further example of a method of providing oxygen to a patient.

DETAILED DESCRIPTION OF EMBODIMENTS

In the following description, numerous specific details are set forth in order to provide a thorough understanding of the present invention. It will be understood by one skilled in the art however, that the present invention may be practiced without some or all of these specific details. In other instances, well known elements have not been described in detail in order not to unnecessarily obscure the description of the present invention.

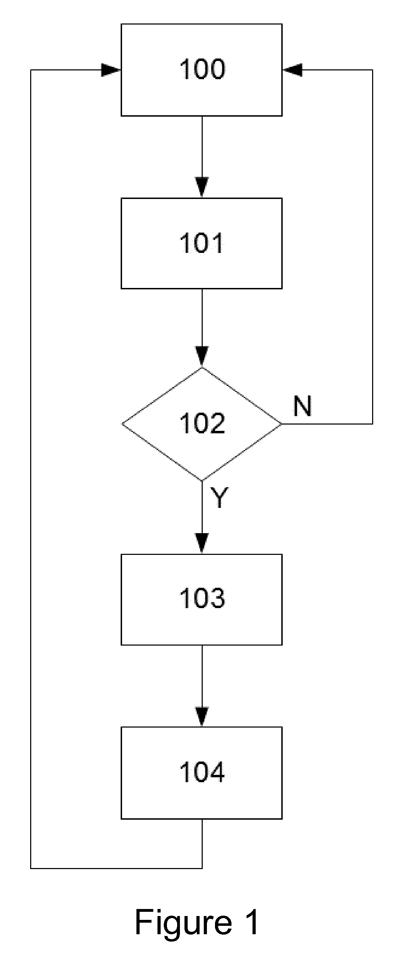

Figure 1 is a flow chart schematically illustrating a first example of a method of providing oxygen to a patient. The method is initiated at step 100, where a level of oxygen in the patient's blood is determined. Afterwards, at step 101, a parameter representative of oxygen insufficiency may be determined by comparing the determined level of oxygen in blood (from step 100) with an oxygen level threshold. Step 102 verifies whether there is oxygen insufficiency or not. It will be clear that in an alternative representations, steps 101 and 102 could be combined into a single step.

If the oxygen in blood is considered insufficient Y, the method continues to step 103, where a need of oxygen is determined depending at least on the parameter representative of oxygen insufficiency (from step 101). Then, at step 104, a continuous supply of the oxygen need (from step 103) to the patient is caused until the oxygen need is determined to be substantially null.

If, at step 102, the determined oxygen in blood is not considered insufficient N, the method continues back to the initial step 100 to initiate a new iteration of the method. After initiating the continuous supply of oxygen to the patient, at step 104, the method continues back to the initial step 100 to initiate a new iteration of the method. When the method returns to the initial step 100, this step 100 may be immediately executed or not depending on when patient's blood will be analysed to determine the oxygen in blood again. Blood analysis may be performed under a predefined frequency.

Therefore, the step 100 of determining the level of oxygen in patient's blood may depend on the abovementioned frequency, such that an iteration of the method may be (re)initiated every certain time (e.g. every 5 seconds, 10 seconds, 30 seconds or 1 or 2 minutes, depending on said defined frequency). The oxygen need may also be determined satisfied when the step 102 determines in a subsequent iteration that there is no oxygen insufficiency (because the oxygen level is considered acceptable at this moment) although there is a supply of oxygen in progress. In this last case, the ongoing oxygen supply may be interrupted.

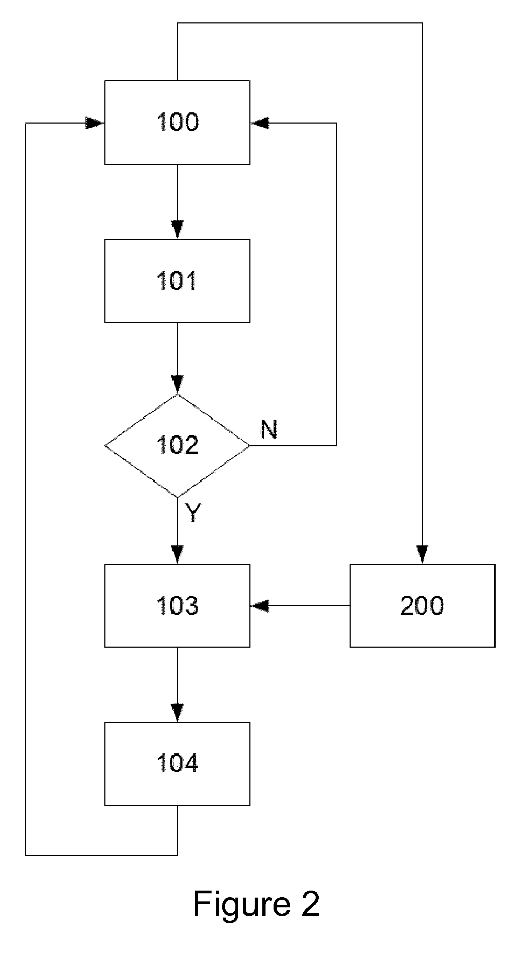

Figure 2 is a flow chart schematically illustrating another example of a method of providing oxygen supply. The example of figure 2 is generally similar to the example shown in Figure 1. One difference is that an additional step 200 of determining one or more other physiological parameters of the patient (in addition to oxygen in blood) is performed. These other physiological parameters may refer to one or more of the following conditions: patient's activity, patient's temperature, patient's skin impedance, patient's cardiac frequency. The step 200 may further comprise receiving a parameter representative of the patient's medical condition, which may indicate e.g. the evolution of the respiratory illness in the patient (initial phase, advanced phase, terminal phase, etc.).

In addition to the parameter oxygen level in blood, some or all of the above mentioned physiological parameters and/or the patient's medical condition may then be taken into account in step 103 for determining the oxygen need. The step 103 may consider different interrelations between the oxygen in blood, some or all of said one or more further physiological parameters and/or the patient's medical condition, to produce a corresponding amount of oxygen (oxygen need) to be provided to the patient (depending on the determined parameters/condition about the patient).

One or more suitable look-up tables may be used to implement the abovementioned interrelations between parameters/conditions and the amount of oxygen to be delivered. For example, a first of these tables may comprise an "input" column for each parameter/condition (including at least the oxygen in blood) and an "output" column of oxygen insufficiency (depending on the values or combinations of values included the "input" column(s)). Similarly, a second of these tables may comprise an "input" column of oxygen insufficiency and an "output" column of oxygen need.

In a particular example only considering the parameter of the oxygen level in blood, a row of the first table could contain an "input" level of oxygen in blood of 86% and an "output" parameter representative of oxygen insufficiency of 4% (4 percentage points), and a row of the second table could contain an "input" parameter representative of oxygen insufficiency of 4% and an "output" oxygen need of 1 litre of oxygen. Therefore, a level of oxygen in blood of 86% would produce an oxygen need of 1 litre of oxygen when considering such an exemplary configuration of look-up tables and their respective content.

The skilled person in the art will appreciate that different look-up tables configurations and contents may be used depending on the circumstances. For example, a single look-up table comprising all the necessary input/output columns previously commented could be used in step 103 to determine the oxygen need.

The following table 1 refers to a single exemplary look-up table which could be used in step 103 for determining an initial oxygen need (fourth column) from the parameters oxygen in patient's blood (first column), patient's activity (second column), and patient's temperature (third column). The first column could alternatively be the oxygen insufficiency making the comparison with a threshold explicit. This table also includes a further column (fifth column) indicating a special action to undertake if the corresponding values of the "input" parameters denotes or may denote e.g. a critical situation.

In table 1, the sign "-" refers to no activity, the sign "+" refers to a minimum movement, the sign "++" refers to slow walking, and the sign "+++" refers to fast walking. An equivalent of "+" (minimum movement) in cardiac frequency (CF) could be CF < 80 bpm (beats per minute), an equivalent of "++" could be 80 bpm < CF < 100 bpm, and an equivalent of "+++" could be CF > 100 bpm. In an alternative table, the (second) column of "activity" could thus be defined in terms of CF and bpm.

Table 1

| oxygen in blood | activity | temperature | oxygen need | special action |
|---|---|---|---|---|
| > 90% | + or - | +/- | 0 litres | |
| = 90% | - | < 37,5° | 0 litres | |
| = 90% | - | > 37,5° | 1 litre | |
| = 90% | ++ | < 37,5° | 1 litre | |
| < 90% | ++ or +++ | < 37,5° | 2 litres | |
| < 90% | - | > 37,5° | 2 litres | |

If after a certain time period (e.g. 1 minute), a continued oxygen insufficiency is detected, an oxygen supply may be correspondingly increased, for example, in accordance with table 2.

Table 2

| oxygen in blood | activity | temperature | oxygen need | special action |
|---|---|---|---|---|
| < 90% | + | < 37,5° | 1 litre | |
| < 90% | - | > 37,5° | 1 litre | |
| < 90% | + | > 37,5° | 2 litres | |
| < 90% | ++ or +++ | < 37,5° | 2 litres | |
| < 90% | + or - | | 5 litres | send alarm and maintain maximum flow |

The method of Figure 2 thus determines the oxygen need from a "direct" interrelation between different "input" parameters (e.g. first to third columns of the exemplary table 1) and different "output" oxygen volumes and special actions (e.g. fourth and fifth columns of the exemplary tables 1 and 2).

If the oxygen flow has been incrementally increased up to 5 l/min and the oxygen insufficiency still maintains, an alarm may be sent to a control system (of e.g. a HIS), while the maximum flow may be maintained. At the HIS, receipt of such an alarm may trigger contacting the patient or family members.

With reference to table 1 it may be seen that oxygen may be supplied also if the oxygen level is determined to be substantially equal to a predefined threshold, in this case 90%. For example, in case of an oxygen level of 90% and a high temperature and/or alternatively a relatively high activity level, an oxygen supply may start. Step 102 may thus be slightly modified (not only an oxygen insufficiency can lead to an oxygen supply, i.e. oxygen level is equal to or less than threshold).

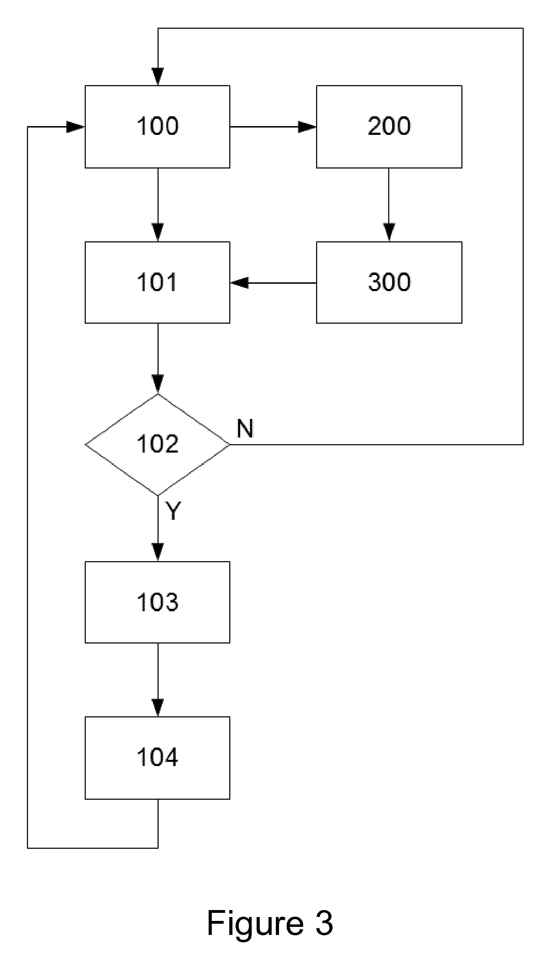

Figure 3 is a flow chart schematically illustrating a third example of a method provided by the invention. This method is generally also similar to the one illustrated by Figure 1. One difference is that the method of Figure 3 further comprises the steps 200 and 300. The step 200 of Figure 3 has the same role as the step 200 of Figure 2, but in this case the obtained physiological parameters and/or medical condition of the patient are used for a different purpose. In this case, the parameters/conditions from step 200 are used in step 300 for accordingly varying the oxygen level threshold used in step 101. This step 101 compares the oxygen in blood (from step 100) with said threshold varied depending on the parameters/condition from step 200 to determine the oxygen insufficiency, which is used in step 103 to determine the oxygen need to be provided to the patient.

In the method of Figure 3, an "indirect" approach is applied in the sense of that no "direct" relations between the parameters/condition (from step 200) and corresponding oxygen needs are used. Instead of this, these parameters/conditions are used for varying the oxygen level threshold, and the oxygen need is determined depending on said varied threshold. This variation of the oxygen level threshold may be obtained by using one or more look-up tables similar to the previous tables 1 and 2, but, in this case, implementing relations between the "input" parameters/condition (from step 200) and corresponding "output" variations of the threshold.

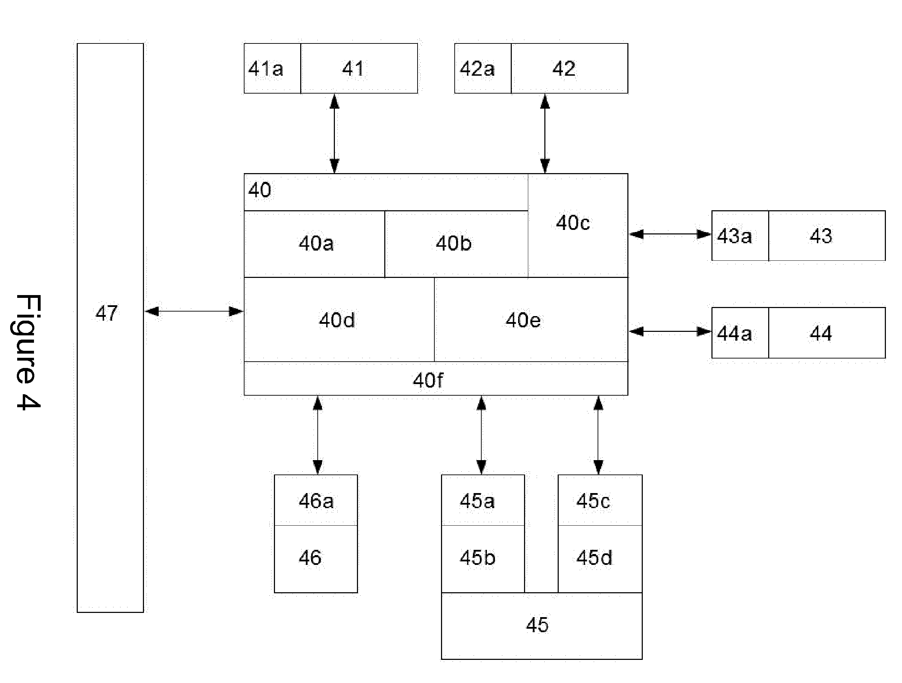

Figure 4 is a block diagram schematically illustrating an example of a system provided by the invention. This embodiment is shown comprising a control unit 40, a plurality of sensors 41 - 44 and 46, and an oxygen provider 45. A further control unit 47 is also shown, which may be e.g. a Hospital information system (HIS). The control unit 40 may comprise different modules, such as e.g. a memory 40a, sensor manager 40b, process manager 40c, communications device/manager 40d, alarm manager 40e, etc.

Each sensor 41 - 44 and 46 may comprise a respective communications device/manager 41 a - 44a and 46a for interchanging any type of signals/data with the control unit 40 through its communications device/manager 40d. These communications may be performed through e.g. a communications network, such as e.g. Internet or a body area network for local communications. Any of the sensors 41 - 44 and 46 may thus send e.g. measurements of the patient to the control unit 40, for the control unit 40 to determine the level of oxygen in patient's blood and possibly other optional parameters (e.g. level of activity, temperature, cardiac frequency, etc.). And the control unit 40 may send corresponding commands to any of the sensors 41 - 44 and 46, such as e.g. commands for the sensor to initiate taking measurements of the patient's body.

The oxygen provider 45 may comprise one or more valves 45b, 45d with respective communications devices/managers 45a, 45c for interchanging signals/data with the control unit 40 through its communications device/manager 40d. Any of the valves 45b, 45d may thus send to the control unit 40 signals indicating e.g. that a given provision of oxygen need has been completed, for the control unit 40 to determine e.g. that said oxygen need has been satisfied. Similarly, the control unit 40 may send corresponding commands to any of the valves 45b, 45d, such as e.g. commands for the valve to initiate supply of a determined oxygen need.

The control unit 40 may also use its communications device/manager 40d for interchanging signals/data with the HIS 47. For example, the control unit 40 may receive from the HIS 47 data about e.g. the medical condition of the patient, and the control unit 40 may send to the HIS 47 data about e.g. an alarm to be activated by the HIS 47.

The control unit 40 may use its memory 40a for e.g. storing measurements received from the sensors 41 - 44 and 46, patient's medical condition received from the HIS 47, or any other data to be used by the control unit 40 for carrying out one or more of the embodiments of the method (provided by the invention). The process manager 40c of the control unit 40 may have implemented corresponding (computer) commands suitable for the system to perform any of the methods explained in other parts of the description.

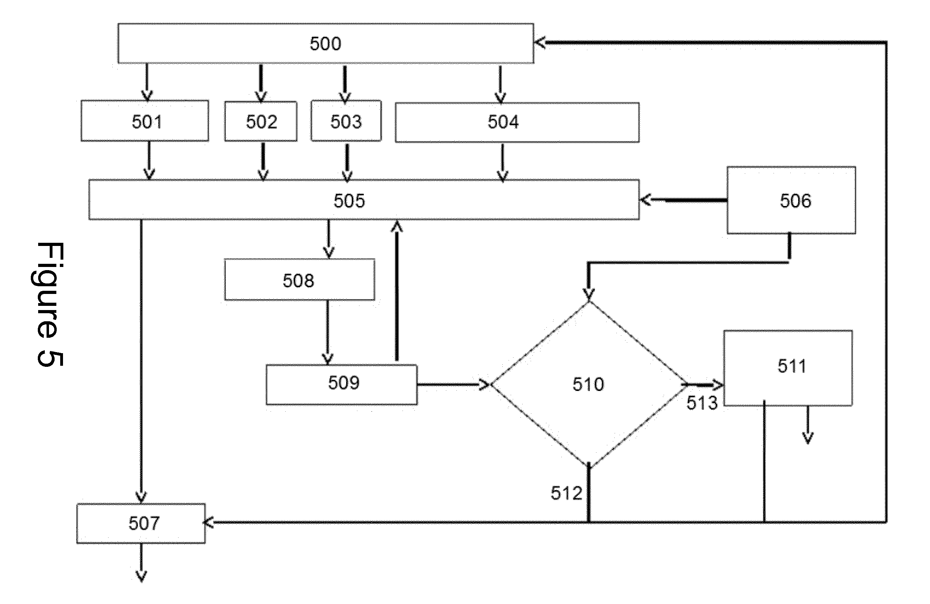

Figure 5 illustrates a further example of providing oxygen which may be implemented in some of the hereinbefore described systems.

In a first stage 500, various measurements may be taken which in this example include the oxygen level in the blood 501, the patient's activity 502, skin temperature 503 and optionally other parameters 504, such as Respiratory Rate (RR), Heart Rate (HR), Heart Rate Variation (HRV) and Thoracic Volume Variation (TVV). These are various parameters 504 which are indicative of either respiration or heart activity.

Based on these measurements 501 - 504 and based on patient data 506, an oxygen need may be determined 505. One suitable example of performing this type of control would be to set an oxygen level threshold based on patient data 506 (including e.g. patient history, recent symptoms, medication etc.) and to determine a need of oxygen based on the error value between the current oxygen level and the oxygen threshold. A PID control may thus be implemented. The gains (constants) in the PID control may be varied based on the measured parameters indicative of patient activity, skin temperature etc.

The flow valve of an oxygen source may be controlled accordingly 507 to deliver the determined oxygen need.

The oxygen need may be stored in a memory 508 and from that point on may form part of the patient's medical history 509. The evolution of the patient's oxygen level (or insufficiency) may be determined 510 and if the oxygen need stays within predetermined acceptable levels 512, the oxygen supply is controlled accordingly 507 and no further action is taken. Based on a constant evaluation of the oxygen level, an oxygen insufficiency may also be anticipated.

If however, the oxygen need (or insufficiency) is outside predefined acceptable levels 513, an alarm may be generated 511. In accordance with the example shown in figure 5, the predetermined acceptable levels of oxygen need or insufficiency may depend on specific patient data 506.

Although only a number of particular embodiments and examples of the invention have been disclosed herein, it will be understood by those skilled in the art that other alternative embodiments and/or uses of the invention and obvious modifications and equivalents thereof are possible. Furthermore, the present invention covers all possible combinations of the particular embodiments described. Thus, the scope of the present invention should not be limited by particular embodiments, but should be determined only by a fair reading of the claims that follow.

**Claims**

1.  System for providing oxygen to a patient, comprising:

    a sensing device having an oxygen sensor for obtaining one or more measurements of a level of oxygen in the patient's blood;
    an oxygen provider for providing oxygen to the patient;
    a control unit for:

    receiving the level of oxygen in blood from the oxygen sensor;
    determining a parameter representative of oxygen insufficiency by comparing the level of oxygen in blood with an oxygen level threshold;
    determining an oxygen need depending at least on the parameter representative of oxygen insufficiency; and
    causing the oxygen provider to continuously provide the oxygen need to the patient until the oxygen need is determined to be substantially null.

2.  System according to claim 1, further comprising:

    one or more further sensors for obtaining one or more measurements of one or more parameters representative of the patient's activity and/or the patient's temperature and/or the patient's skin impedance and/or the patient's cardiac frequency;
    wherein:
    the control unit is further configured to receive the one or more parameters representative of the patient's activity and/or the patient's temperature and/or the patient's skin impedance and/or the patient's cardiac frequency from the one or more further sensors, and
    to determine the oxygen need at least partly based also on the one or more parameters representative of the patient's activity and/or the patient's temperature and/or the patient's skin impedance and/or the patient's cardiac frequency.

3.  System according to claim 2, wherein at least one of the further sensors is also comprised in the sensing device.

4.  System according to any of claims 1 to 3, wherein the sensing device is a percutaneous sensing device.

5.  System according to any of claims 1 to 4, wherein the sensing device is adapted to be arranged in a position of the patient's chest.

6.  System according to any of claims 1 to 5, wherein the control unit is also comprised in the sensing device.

7. System according to any of claims 1 to 6, wherein the control unit is wirelessly connected with the oxygen provider.

8. System according to any of claims 1 to 7, wherein the oxygen provider is a portable oxygen provider.

9. Method of providing oxygen to a patient, comprising:

determining a level of oxygen in the patient's blood;
determining a parameter representative of oxygen insufficiency by comparing the level of oxygen in blood with an oxygen level threshold;
determining an oxygen need depending at least on the parameter representative of oxygen insufficiency;
continuously providing the oxygen need to the patient until the oxygen need is determined to be substantially null.

10. Method according to claim 9, further comprising:

determining a parameter representative of the patient's activity; wherein:

the oxygen need is determined at least partly based also on the parameter representative of the patient's activity.

11. Method according to any of claims 9 or 10, further comprising:

determining a parameter representative of the patient's skin temperature; wherein:

the oxygen need is determined at least partly based also on the parameter representative of the patient's skin temperature.

12. Method according to any of claims 9 to 11, further comprising:

determining a parameter representative of the patient's skin impedance; wherein:

the oxygen need is determined at least partly based also on the parameter representative of the patient's skin impedance.

13. Method according to any of claims 9 to 12, further comprising:

determining a parameter representative of the patient's cardiac frequency; wherein:

the oxygen need is determined at least partly based also on the parameter representative of the patient's cardiac frequency.

14. Method according to any of claims 9 to 13, further comprising:

receiving a parameter representative of the patient's medical condition; wherein:

the oxygen need is determined at least partly based also on the parameter representative of the patient's medical condition.

15. Method according to any of claims 9 to 14, wherein determining the oxygen need comprises using a PID control loop based on at least one of the representative parameters.

Figure 1

Figure 2

Figure 3

Figure 4

Figure 5

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

EP 14 38 2007

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2002/112726 A1 (SCHMIDT MATTHEW F [US] ET AL) 22 August 2002 (2002-08-22)<br>* paragraph [0044] - paragraph [0121]; figures 1-4 * | 1,2,4 | INV.<br>A61M16/06<br><br>ADD.<br>A61M16/00 |
| X | US 2010/083968 A1 (WONDKA ANTHONY D [US] ET AL) 8 April 2010 (2010-04-08)<br>* paragraph [0054] - paragraph [0086]; figures 1-7,11 * | 1-8 | |
| X | US 2010/116270 A1 (EDWARDS PAUL L [US] ET AL) 13 May 2010 (2010-05-13)<br>* paragraph [0042] - paragraph [0055]; figures 1-3 *<br>* paragraph [0155] - paragraph [0171]; figures 7,8,19-21 * | 1-8 | |
| X | US 2006/124128 A1 (DEANE GEOFFREY F [US] ET AL) 15 June 2006 (2006-06-15)<br>* paragraph [0020] - paragraph [0038]; figures 1-6B * | 1,5,7,8 | |

-/--

**TECHNICAL FIELDS SEARCHED (IPC)**

A61M

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| The Hague | 27 August 2014 | Zeinstra, Hilaire |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................................
& : member of the same patent family, corresponding document

EPO FORM 1503 03.82 (P04E07)

Europäisches
Patentamt

European
Patent Office

Office européen
des brevets

**PARTIAL EUROPEAN SEARCH REPORT**

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | FR 2 835 188 A1 (BEAR MEDICAL [FR]) 1 August 2003 (2003-08-01) * page 3, line 10 - page 6, line 13; figure 1 * ----- | 1-3,8 | |
| X | FR 2 917 978 A1 (BEAR MEDICAL SOC PAR ACTIONS S [FR]) 2 January 2009 (2009-01-02) * page 3, line 17 - page 8, line 9; claims 1,3,4; figures 1-2 * ----- | 1-8 | |
| | | | TECHNICAL FIELDS SEARCHED (IPC) |

EPO FORM 1503 03.82 (P04C10)

2

Europäisches
Patentamt

European
Patent Office

Office européen
des brevets

**INCOMPLETE SEARCH
SHEET C**

**Application Number**

EP 14 38 2007

Claim(s) completely searchable:
      1, 3-8

Claim(s) not searched:
      9-15

Reason for the limitation of the search (non-patentable invention(s)):

Independent claim 9 and dependent claims 10 - 15 define a method for providing oxygen to a patient (administering a medication to a patient for the treatment of a mammal: therapeutic effect). According to Article 53(c) EPC, the subject matter of these claims is not regarded as patentable (Method for treatment of the human body by therapy).

      -----

Further limitation of the search

Claim(s) completely searchable:
      1, 3-8

Claim(s) searched incompletely:
      2

Reason for the limitation of the search:

The present claim 2 relates to an extremely large number of possible systems with the repetitive use of the wording "and/or". Support and disclosure within the meaning of Articles 84 and 83 EPC are to be found, however, for only a very small proportion of the system claimed. Non-compliance with the substantive provisions is such that a meaningful search of the whole claimed subject-matter of the claim can not be carried out (Rule 63 EPC and Guidelines B-VIII, 3).

**EP 2 893 948 A1**

ANNEX TO THE EUROPEAN SEARCH REPORT
ON EUROPEAN PATENT APPLICATION NO.

EP 14 38 2007

This annex lists the patent family members relating to the patent documents cited in the above-mentioned European search report.
The members are as contained in the European Patent Office EDP file on
The European Patent Office is in no way liable for these particulars which are merely given for the purpose of information.

27-08-2014

| Patent document cited in search report | | Publication date | Patent family member(s) | | Publication date |
|---|---|---|---|---|---|
| US 2002112726 | A1 | 22-08-2002 | US | 6371114 B1 | 16-04-2002 |
| | | | US | 2002112726 A1 | 22-08-2002 |
| | | | US | 2003145852 A1 | 07-08-2003 |
| | | | US | 2004159323 A1 | 19-08-2004 |
| | | | US | 2006213519 A1 | 28-09-2006 |
| US 2010083968 | A1 | 08-04-2010 | CA | 2739435 A1 | 08-04-2010 |
| | | | EP | 2344791 A1 | 20-07-2011 |
| | | | JP | 2012504473 A | 23-02-2012 |
| | | | US | 2010083968 A1 | 08-04-2010 |
| | | | WO | 2010039989 A1 | 08-04-2010 |
| US 2010116270 | A1 | 13-05-2010 | EP | 2355882 A2 | 17-08-2011 |
| | | | JP | 2012508074 A | 05-04-2012 |
| | | | US | 2010116270 A1 | 13-05-2010 |
| | | | WO | 2010054323 A2 | 14-05-2010 |
| US 2006124128 | A1 | 15-06-2006 | US | 2006124128 A1 | 15-06-2006 |
| | | | WO | 2006053272 A1 | 18-05-2006 |
| FR 2835188 | A1 | 01-08-2003 | FR | 2835188 A1 | 01-08-2003 |
| | | | WO | 03063939 A1 | 07-08-2003 |
| FR 2917978 | A1 | 02-01-2009 | FR | 2917978 A1 | 02-01-2009 |
| | | | WO | 2009019338 A2 | 12-02-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 2002112726 A